(19) Europäisches Patentamt
European Patent Office
Office européen des brevets

(11) **EP 3 375 418 A1**

(12) **EUROPEAN PATENT APPLICATION**

(43) Date of publication:
**19.09.2018 Bulletin 2018/38**

(51) Int Cl.:
***A61F 11/12*** *(2006.01)*

(21) Application number: **18168983.7**

(22) Date of filing: **06.05.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.05.2014 US 201462000881 P**

(62) Document number(s) of the earlier application(s) in accordance with Art. 76 EPC:
**15724856.8 / 3 145 462**

(71) Applicant: **3M Innovative Properties Company St. Paul, MN 55133-3427 (US)**

(72) Inventor: **Cai, Feng Saint Paul, MN 55133-3427 (US)**

(74) Representative: **Herzog, Fiesser & Partner Patentanwälte PartG mbB Isartorplatz 1 80331 München (DE)**

Remarks:
This application was filed on 24-04-2018 as a divisional application to the application mentioned under INID code 62.

(54) **TETHER FOR PERSONAL PROTECTIVE DEVICE, SUCH AS A HEARING PROTECTION DEVICE**

(57) A personal protective device tether. The tether comprises an elongate tether. The tether defines opposing, first and second ends and a length separating the first and second ends. The tether is configured to provide an initial state in which the length is an initial length (L1). Further, the tether is configured to self-retain a stretched state when stretched from the initial state at a first temperature (T1), the length in the stretched state being a stretched length (L2) that is greater than Ll. Even further, the tether is configured to self-transition from the stretched state to a recovered state when heated to a second temperature (T2) greater than T1.

Fig. 3

**Description**

**Technical Field**

The present disclosure relates to personal protective devices. More particularly, it relates to a tether for a personal protective device, such as a tether connected to and maintaining two earplugs.

**Background**

Various personal protective devices make use of or incorporate a tether or flexible cord. For example, eyewear, hard hats, respirators, communication devices, etc., all oftentimes include a tether that promotes wearing and/or storage of the device by a user. Hearing protective and noise attenuating devices also can commonly include a tether. The use of hearing protective and noise attenuating devices is well known, and various types of devices are available including, but not limited to, ear muffs, semi-aural devices, and earplugs. Earplugs are particularly preferred for their effectiveness in attenuating sound and for comfort properties provided thereby.

An earplug generally comprises a sound attenuating element that is placed in the ear canal of a wearer to provide desired sound attenuation. The sound attenuating element is commonly made of a resilient foam material or a flexible, rubber-like material.

Several types of earplugs are known. A roll-down type earplug typically has a resilient foam body which is rolled by a user to reduce a diameter thereof. A portion of the reduced diameter earplug body is then inserted in the ear canal and allowed to expand therein to fill the canal and provide desired attenuation. The remaining portion of the earplug body extends or projects from the ear canal and provides a handle for removing the plug. Alternatively, a push-to-fit stem type earplug includes a resilient body having a rigid or semi-rigid stem embedded therein and, most typically, extending therefrom. The stem provides a degree of rigidity to the earplug that facilitates insertion of the plug body into the ear canal. During use, the exposed stem portion of the push-to-fit stem earplug extends or projects from the ear canal, thus providing a handle for removal of the earplug.

Earplugs often include a tether or cord that attaches a pair of plugs and extends therebetween. The tether allows a user to hang the earplugs around their neck or elsewhere when the plugs are not being used. Also, the tether permanently relates a pair of earplugs and prevents against loss thereof. In the context of other tethered personal protective devices, the provided tether oftentimes performs these same functions.

Conventional tethers are commonly designed so that the tether may be permanently attached to the earplugs and more specifically, each end of the tether may be permanently attached to the ends of the earplugs. In the case of roll-down type earplugs, a tether is attached to one end of the resilient body. Push-to-fit stem type earplugs generally have the tether attached at the exposed portion of the stem. Tethers may be attached to the various types of earplugs, for example, by ultrasonic welding or by adhesive bonding.

Regardless of how the tether is attached to the earplugs, tethered earplug pairs (or other tethered personal protective devices) are often sold in individual packages. For example, a single pair of earplugs attached by a tether are enclosed in a small plastic sealed package. A user acquires the package and opens the same to access the tethered earplug pair. In this way, an individual pair of earplugs is conveniently provided to a user while a sanitary condition of the plugs in the sealed package is maintained.

From a mass production perspective, manufacturers of tethered earplugs (or other tethered personal protective devices) endeavor to select a standard size or length for the tether that best meets the expected needs of most users. A common length for the tether of a tethered earplug device is 24 inches (61 cm), and is expected to be more than sufficiently-long for most users (e.g., will accommodate "large" distances between ears due to head size, hair style, wearing of head gear, etc.). While viable, packaging of tethered earplugs with a longer than necessary tether requires additional packaging space and entails more complex processes. Further, the standard length selected by the manufacturer may be too short for some users.

Efforts have been made to provide a tethered earplug product with an expandable or stretchable tether. For example, US Patent No. 5,668,354 to Falco discloses a one-time stretchable ribbon connecting two earplugs. US Application Publication No. 2005/0230181 to Woo discloses a cord connecting two earplugs that can be stretched by a user-applied force on the order of 15 pounds.

In light of the above, any improvements to length-adjustable tethered personal protective devices, such as tether earplugs, would be well received.

**Summary**

Some aspects in accordance with principles of the present disclosure relate to a tether for a personal protective device. The tether includes an elongate tether comprising a polymer, optionally a polymer blend. The tether defines opposing, first and second ends, as well as a length separating the opposing ends. The tether is configured to provide an initial state in which the length is an initial length (L1). The tether is further configured to self-retain a stretched state when stretched from the initial state at a first temperature (T1). The length in the stretched state is a stretched length (L2) that is greater than the initial length (L1). The tether is further configured to self-transition from the stretched state to a recovered state when heated to a second temperature (T2). The second temperature (T2) is greater than the first temperature (T1). The length in the recovered state is a recovered length (L3) that is less than the stretched length (L2). With this construction, the tether can be stretched by a user to the stretched state in which the stretched length (L2) approximates the length desired by the user; following use, the tether can be contracted to the recovered length (L3) for storage. Or, if the stretched length (L2) is longer than desired by the user, the tether can be contracted to a desired length in the recovered state (i.e., the recovered length (L3)) and then worn. In some embodiments, the tether is capable of being stretched to a stretched length on the order of at least 5x the initial length at a user-applied tensile force of not more than 5 lbs (22 N). In other embodiments, the tether is capable of being transitioned to the recovered state when heated from room temperature to a second temperature (T2) on the order of 60 °C - 70 °C (e.g., placed under a stream of hot water). In other embodiments, the polymer blend includes at least two thermoplastic elastomeric polymers, for example ethylene vinyl acetate (EVA) and thermoplastic polyurethane (TPU).

Other aspects of the present disclosure relate to a tethered personal protective device including a tether and a personal protective assembly. The tether includes a polymer, optionally a polymer blend, and defines opposing first and second ends separated by a length as described above. The personal protective assembly is attached to the first and second ends of the tether. In some embodiments, the personal protective assembly includes first and second earplugs, with the first earplug attached to the first end of the tether, and the second earplug attached to the second end of the tether.

Yet other aspects of the present disclosure relate to a method of using a personal protective device. The method includes receiving a personal protective device including an elongate tether. The tether comprises a polymer, optionally a polymer blend, and defines opposing, first and second ends, and a length separating the first and second ends. The personal protective device is received with the tether in an initial state in which the length is an initial length (L1). A stretching force is applied to the tether at a first temperature (T1) to transition the tether to a stretched state. In the stretched state, the length of the tether is a stretched length (L2) that is greater than the initial length (L1). The tether self-retains the stretched state. The personal protective device is optionally worn by a user with the tether in the stretched state. The tether is heated to a second temperature (T2) that is greater than the first temperature (T1), with the tether self-transitioning form the stretched state to a recovered state. In the recovered state, the length of the tether is a recovered length (L3) that is less than the stretched length (L2). The personal protective device is optionally stored or worn by a user with the tether in the recovered state. In some embodiments, the tether can be repeatedly stretched to differing stretched states (and thus stretched lengths), and repeatedly caused to self-transition to differing recovered states (and thus recovered lengths).

Yet other aspects of the present disclosure relate to a packaged personal hearing protection article. The packaged article includes a personal hearing protection device and a sealed package. The personal hearing protection device includes an elongate tether, a first earplug and a second earplug. The tether comprises a polymer, optionally a polymer blend, and defines opposing first and second ends, and a length separating the first and second ends. The tether is configured to provide an initial state in which the length is an initial length (L1). The tether is further configured to self-retain a stretched state when stretched from the initial state at a first temperature (T1). The length in the stretched state is a stretched length (L2) that is greater than the initial length (L1). The tether is further configured to self-transition from the stretched state to a recovered state when heated to a second temperature (T2). The second temperature (T2) is greater than the first temperature (T1). The length in the recovered state is a recovered length (L3) that is less than the stretched length (L2). The first earplug is attached to the first end of the tether, and the second earplug is attached to the second end of the tether. The personal hearing protection device is contained within the sealed package.

**Brief Description of Drawings**

FIG. 1 is a perspective view of a tethered personal protective device, including a tether in accordance with principles of the present disclosure;
FIG. 2 is an enlarged side view of a portion of the tether of FIG. 1 in an initial state;
FIG. 3 is an enlarged side view of a portion of the tether of FIG. 1 in a stretched state;
FIG. 4 is an enlarged side view of a portion of the tether of FIG. 1 in recovered state; and
FIG. 5 is a perspective, exploded view of a packaged tethered personal protection device article in accordance with principles of the present disclosure.

**Detailed Description**

The present disclosure provides a tether for a personal protective device, for example personal hearing protection device such as a pair of earplugs. The tether is an elongate body defining opposing ends and a length separating the ends. The tether comprises a polymer blend in some embodiments. An exemplary tether can be stretched from an initial length to a stretched length, and will self-maintain the stretched length. Further, when heated, an exemplary tether will self-transition or self-contract from the stretched length to a recovered length. An exemplary tether according to the present disclosure allows a user to select a desired length of the tether, through various stretching and/or recovery operations, for wearing, storing, etc., the corresponding personal protective device.

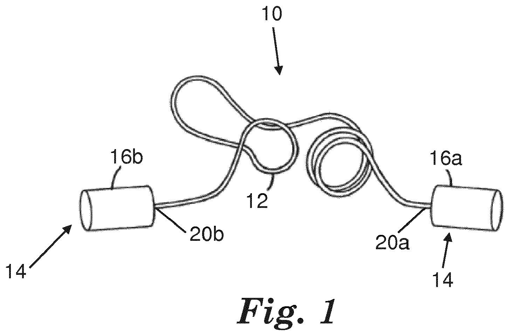

One embodiment of an exemplary tethered personal protective device 10 in accordance with principles of the present disclosure is shown in FIG. 1. The personal protective device 10 includes a tether 12 and a personal protective assembly 14 (referenced generally). Details on the various components are provided below. In general terms, however, the personal protective assembly 14 can assume a wide variety of forms, and in some embodiments is a hearing protection device including first and second hearing protection members 16a, 16b. The hearing protection members 16a, 16b can be earplugs. Other formats that may or may not include two discrete components are equally acceptable (e.g., other hearing protection devices, eyewear, hard hat, respirator, communication device, etc.). Regardless, the tether 12 is attached to the personal protective assembly, and is stretchable by a user from an initial state to a stretched state in which a length of the tether 12 is increased, and can be shortened from the stretched state to a recovered state in which a length of the tether 12 is decreased (as compared to the length in the stretched state).

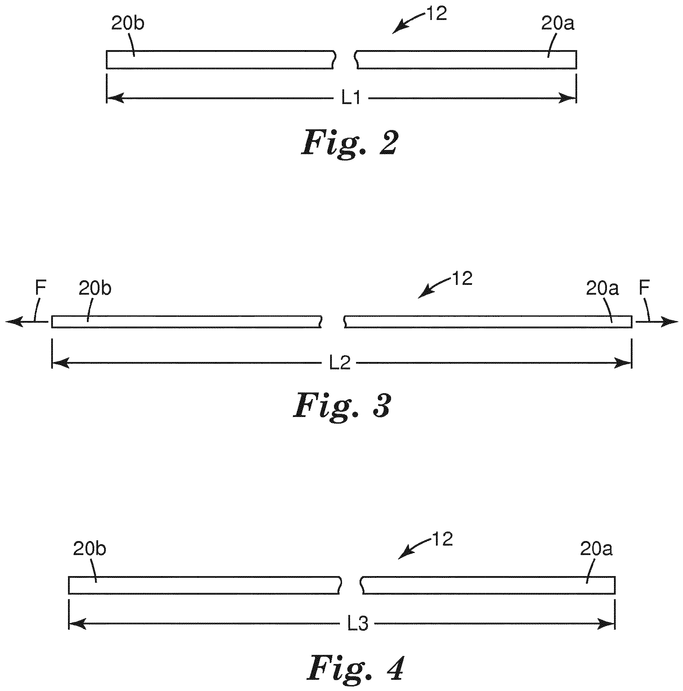

As implicated by the above explanations, the tether 12 can be described as having or defining a length, and is configured to exhibit or maintain various states. FIG. 1 reflects the tether 12 in the initial state, with the "initial state" being in reference to the tether 12 (or the personal protective device 10 as a whole) as initially provided to a user. The tether 12 is a flexible body, and defines opposing, first and second ends 20a, 20b. The first and second ends 20a, 20b are separated by a length of the tether 12. The "length" of the tether 12 is in reference to a linear length of the tether 12 from the first end 20a to the second end 20b, regardless of a shape to which the tether 12 is coiled or flexed. Stated otherwise, for ease of illustration, FIG. 1 depicts the tether 12 partially coiled on to itself. The "length" of the tether 12 is the continuous, physical length of the tether 12, and is not necessarily the linear distance between the opposing ends 20a, 20b (e.g., in the partially coiled arrangement of FIG. 1, the linear distance between the opposing ends 20a, 20b is less than the length of the tether 12). The "length" of the tether 12 can be determined by straightening the tether 12 (e.g., the tether 12 is made taut) and measuring the distance between the opposing ends 20a, 20b as generally reflected by FIG. 2. FIG. 2 represents the tether 12 in the initial state, having an initial length L1. The initial length L1 can vary as function of the particular personal protective assembly 14 (FIG. 1) and in accordance with a length viewed as being desirable by an expected end user. For example, with non-limiting embodiments in which the personal protective assembly 14 is the pair of earplugs 16a, 16b, the initial length L1 of the tether can optionally be in the range of 12 - 36 inches (30 - 91 cm), alternatively in the range of 18 - 30 inches (46 - 99 cm), alternatively on the order of 24 inches (61 cm). Other initial lengths L1 are also acceptable when the tether 12 is employed with the pair of earplugs 16a, 16b. Further, with embodiments in which the tether 12 is used with other personal protective assembly formats, the initial length L1 can be within the ranges described above, or can be longer or can be shorter.

The tether 12 can be stretched (and repeatedly re-stretched) by a user from the initial state to a stretched state as generally reflected by FIG. 3. As a point of reference, the tether 12 is configured to provide multitude of different stretched states, with the particular stretched state being selected by a user as described below and characterized by the tether 12 self-maintaining the stretched state without failure (i.e., the tether 12 remains continuous and is not severed). Regardless, in the stretched state, the tether 12 has a stretched length L2 that is greater than the initial length L1 (FIG. 2). For example, the stretched length L2 can be at least 1.5x, alternatively at least 2x, alternatively at least 4x, alternatively at least 5x, alternatively at least 8x the initial length L1. For example, where the initial length L1 is 24 inches (61 cm), the tether 12 can be configured to be stretched, without failure, to a stretched length of at least 96 inches (244 cm) or more.

The tether 12 is configured to be stretched from the initial state to the stretched state by a user-applied tensile force (designated by the arrows "F" in FIG. 3) at or adjacent the opposing ends 20a, 20b. In some embodiments, the stretched states described above (e.g., the stretched length L2 being at least 1.5x, alternatively at least 2x, alternatively at least 4x, alternatively at least 5x, alternatively at least 8x, the initial length L1) are achieved by a user-applied tensile force F of not more than 5 lbs, alternatively not more than 4 lbs under temperature conditions of not more than 50 °C, alternatively not more than 40 °C. For example, in some embodiments, the exemplary tether 12 is configured to be successfully transitioned by a user-applied tensile force on the order of 5 lbs applied with the tether 12 at room temperature to a stretched length L2 that is at least 5x the initial length.

As described in greater detail below, in some embodiments the tether 12 is comprised of a polymer blend of thermoplastic elastomeric polymers that may, in some constructions, exhibit an inherent, slight recovery or shortening upon removal of the stretching or tensile force F. The phrase "stretched state" as used in the present disclosure is in reference to a state of the tether 12 upon the completion of a user-prompted stretching operation, including removal of the applied stretching or tensile force (e.g., a user grasps the tether 12 with his or her hands at or adjacent the opposing ends 20a, 20b, manually pulls the ends 20a, 20b away from one other to cause the tether 12 to stretch, and then releases the tether 12). The exemplary tethers 12 of the present disclosure self-maintain the stretched state, and exhibit limited, if any, contraction or recovery upon removal of the user-applied stretching or tensile force F. Thus, the stretched length L2 (i.e., the length of the tether 12 following removal of the stretching or tensile force F) as self-maintained by the tether 12 is close to the length as imparted by the user-applied force (i.e., immediately prior to removal of the stretching or tensile force F). The ability of the tether 12 to self-retain the stretched length L2 that is relatively the same as the imparted length is referred to as "fixity rate" ($R_f$) and is determined by measuring the length of the tether 12 immediately prior to removing the stretching or tensile force, and measuring the length of the tether 12 after a short dwell time (e.g., 3 minutes) following removal of the stretching or tensile force. The fixity rate $R_f$ represents a comparison of fixed strain and maximum strain, and can be expressed as a percentage as:

$$(Eq. 1) \qquad R_f = (\varepsilon_f / \varepsilon_m) \times 100,$$

where:

$\varepsilon_f$ = fixed strain = (L2 - L1)/L1,
$\varepsilon_m$ = maximum strain = (length with applied stretching force - L1)/L1,
L1 = initial length, and
L2 = stretched length (after removal of stretching force).

In some embodiments, the tether 12 is configured to be significantly stretched without failure, and to self-maintain the stretched length with limited self-contraction or self-recovery at normal (e.g., room) temperatures. For example, in some embodiments, the tether 12 is configured to exhibit a fixity $R_f$ of not less than 50%, alternatively not less than 60%, alternatively not less than 70%, and alternatively not less than 80% at a stretched length L2 not less than 4x the initial length L1.

The tether 12 is further configured to self-transition from the stretched state to the recovered state when heated. As generally reflected by FIG. 4, the tether 12 has a recovered length L3 in the recovered state, with the recovered length L3 being less than the stretched length L2. The tether 12 can optionally achieve a multitude of different recovered states (and thus recovered lengths L3) as a function of the starting, stretched length L2, elevated temperature, and length of exposure time to the elevated temperature. In some embodiments, the tether 12 is configured to self-transition to the recovered state when exposed or subjected to an elevated temperature of at least 40 °C, alternatively at least 50 °C, alternatively at least 60 °C, alternatively at least 70 °C, and alternatively in the range of 50 °C - 70 °C. For example, in some embodiments, the tether 12 is configured to self-maintain the stretched state (and thus the stretched length L2) at or about room temperature (e.g., 19 °C - 26 °C), and self-transition to the recovered state when placed in hot water or other environment with a temperature on the order of at least 40 °C.

As described in greater detail below, in some embodiments the tether 12 is comprised of a polymer or a polymer blend of thermoplastic elastomeric polymers that may, in some constructions, exhibit an ability to contract or shrink from the stretched state when heated, and a shape memory attribute that self-retains the shortened arrangement when the source of heat is removed. The phrase "recovered state" as used in the present disclosure is in reference to a state of the tether 12 upon the completion of a heating operation or cycle, including removal of the tether 12 from the elevated temperature condition (e.g., a user exposes the tether 12 to an elevated temperature for a relatively short timed period, and then returns the tether 12 to the temperature environment of normal use (such as room temperature)). The exemplary tethers 12 of the present disclosure self-transition to, and self-maintain, the recovered state. The ability of the tether 12 to self-transition to the recovered length L3 when heated, and retain the recovered length L3 upon removal of the source of heat can be referred to as "recovery rate" ($R_R$) and is determined by measuring the length of the tether 12 immediately prior to exposing the tether 12 to an elevated temperature, and measuring the length of the tether 12 after a short dwell time (e.g., 5 minutes) at room temperature (e.g., 22 °C) following removal of the tether 12 from the elevated temperature environment.. The recovery rate $R_R$ represents the strain experienced by the tether 12 in recovering or contracting back toward the initial length L1, and can be expressed as a percentage in terms of strain following heating as:

$$(Eq. 2) \qquad R_R = [(L2 - L3) / L2] \times 100,$$

where:

L2 = stretched length (after removal of stretching force), and

L3 = recovered length (after heat then dwell).

In some embodiments, the tether 12 is configured to experience significant recovery or contraction when heated. It will be recognized that a calculated recovery rate $R_R$ value for the tether 12 may be dependent, to at least some extent, upon the starting or stretched length L2 with embodiments in which the tether 12 is configured to be significantly stretched without failure. For example, an exemplary tether 12 could provide an initial length L1 of 24 inches (61 cm), and could be successfully stretched to a stretched length L2 anywhere in the range of 50 - 100 inches (127 - 254 cm). A different recovery rate $R_R$ might be determined for this same tether 12 when measured from a starting stretched length of 50 inches (127 cm) as compared to a starting stretched length of 100 inches (254 cm) (e.g., the tether 12 as stretched to 50 inches (127 cm) might have a recovered length of 30 inches (76 cm) and thus a recovery rate $R_R$ of 40%, whereas the same tether 12 stretched to 100 inches (254 cm) and subjected to the same recovery conditions might have a recovered length of 31 inches (79 cm) and thus a recovery rate $R_R$ of 69%). To better characterize a recovery attribute of the tether 12, then, in some embodiments, the recovery rate $R_R$ is determined with the tether 12 first stretched to a stretched length L2 that is at least 4x the initial length L1. Under these circumstances, in some embodiments, the tether 12 is configured to exhibit a recovery rate $R_R$ of at least 50%, alternatively at least 60%, alternatively at least 70%, alternatively at least 80% following exposure to a temperature of not less than 50 °C for a time period of not more than one minute. Alternatively not more.

Another factor indicative of an ability of the tether 12 to self-contract or recover when heated is a shape memory rate $R_S$, and relates the measured recovered length L3 with the initial length L1. More particularly, the shape memory rate $R_S$ represents a comparison of recovery strain and fixed strain, and can be expressed as a percentage as:

$$\text{(Eq. 3)} \qquad R_S = [(\varepsilon_f - \varepsilon_R)/\varepsilon_f] \times 100,$$

where:

   $\varepsilon_f$ = fixed strain (Eq. 1),
   $\varepsilon_R$ = recovery strain = |(L1 - L3)| / L1,
   L1 = initial length (prior to stretch), and
   L3 = recovered length after heat then dwell.

In some embodiments, the exemplary tether 12 exhibits a shape memory rate $R_S$ of at least 60%, alternatively at least 65%, alternatively at least 70%.

Returning to FIG. 1, in some embodiments, the exemplary tethers 12 of the present disclosure are configured to satisfy the performance criteria (e.g., stretching and recovery) described above by forming the tether 12 from a polymer blend or alloy. In some embodiments, the polymer blend or alloy includes at least two different thermoplastic elastomers, such as ethylene vinyl acetate (EVA), thermoplastic polyurethane (TPU), thermoplastic olefin (TPO), and styrene-butadiene copolymers, and that when combined, exhibit physical cross-linking that allows for significant stretching a room temperature and significant self-recovery or contraction at elevated temperatures. It has surprisingly been found that forming the tether 12 from a polymer blend of EVA and TPU results in the tether 12 capable of significant stretching from the initial state to the stretched state at room temperature using minimal tensile force and experiencing minimal contraction (e.g., can be stretched from an initial length L1 of 24 inches to a stretched length L2 of 100 inches (254 cm) in response to a stretching or tensile force of 4-5 lbs (18 - 22 N)), and capable of significant self-recovery from the stretched state to the recovered state when subjected to an elevated temperature of about 60 °C - 70 °C for less than 1 minute (e.g., will recover from the stretched length L2 of 100 inches (254 cm) to a recovered length L3 of 30 inches (76 cm) following exposure to the elevated temperature for less than 1 minute, optionally not more than 30 seconds). In related embodiments, the exemplary polymer blend further balances an ability to promote attachment of the tether 12 with the particular personal protective assembly 14.

A ratio of the thermoplastic elastomer components of the optional polymer blend-based tether 12 can be selected to improve stretching and/or recovery attributes. For example, in some embodiments, it has surprisingly been found that a polymer blend or alloy useful for forming the exemplary tether 12 in accordance with principles of the present disclosure includes 50 wt% - 95 wt% EVA and 5 wt% - 50 wt% TPU; alternatively, the polymer blend includes 60 wt % - 80 wt% EVA and 10 wt% - 30 wt% TPU. In yet other embodiments, the polymer blend useful for forming the exemplary tether includes 66 wt% - 72 wt% EVA and 18 wt% - 22 wt% TPU. It has surprisingly been found that a polymer blend incorporating 68 wt% EVA and 20 wt% TPU can produce well-performing tethers 12 in accordance with principles of the present disclosure. In one non-limiting example where the personal protective assembly 14 includes the pair of earplugs 16a, 16b formed of a foam material, it has surprisingly been found that the above exemplary polymer blends of EVA and TPU promote bonding of the tether 12 to the foam earplugs 16a, 16b with an adhesive (unlike a tether formed from EVA alone that does not readily bond with adhesives conventionally employed with foam earplugs). In this regard, the particular formulation or type of EVA can be important to achieve the beneficial performance characteristics of the present disclosure. In some embodiments, polymers and polymer blends useful for forming the exemplary tethers 12 of the present disclosure incorporate an EVA component that comprises vinyl acetate in the range of 10 - 50%. Other amounts and/or other thermoplastic elastomeric polymer constituents are also envisioned. In yet other embodiments, the exemplary tethers of the present disclosure are optionally formed from a single polymer (that may be combined with other, non-polymer materials).

As implied by the above, the optional polymer blend useful for forming tethers of the present disclosure may include materials or ingredients in addition to the two (or more) thermoplastic elastomers. For example, conventional additives such as lubricants, fillers, pigments, etc., can be included at a combined weight percent of less than 15 wt% in some embodiments.

The exemplary tethers 12 of the present disclosure can assume a wide variety of shapes and forms yet satisfy the performance criteria (e.g., stretching and recovery) described above. For example, the tether 12 can be a solid body, and optionally is tubular. The tether 12 can have various cross-sectional shapes, and in some embodiments is circular in transverse cross-section (in at least the initial state). In other embodiments, the tether 12 can have other cross-sectional shapes (in at least the initial state), such as oval, triangular, rectangular (include square), a complex shape, etc. A transverse cross-sectional size or area of the tether 12 can also assume a wide variety of dimensions in accordance with principles of the present disclosure, and is optionally selected as a function of the particular personal protective assembly 14. For example, where the personal protective assembly 14 includes the pair of earplugs 16a, 16b, the tether 12 can have a cross-sectional diameter (or other major dimension) that is less than a size of the earplugs 16a, 16b, for example on the order of 0.01 - 1 inch (0.25 - 25 mm). Other dimensions are contemplated by the present disclosure and are also acceptable.

In some embodiments, the tether 12 can be formed by conventional polymer extrusion processing. For example, single screw extrusion, twin screw extrusion, batch mixing, etc. In more general terms, with embodiments in which the tether 12 is formed from a polymer blend, the thermoplastic elastomeric polymer components can be provided in pellet form and dry mixed with any other, optional powder ingredients. The mixture is fed to a melt mixer (continuous or batch) operated at a requisite operating temperature. The constituents are melt mixed and blending at the requisite temperature until a desired degree of mixing is achieved. The resultant mixture is discharged a size reduce into pellet form. The pellet is dried in a conventional dryer. The polymer blend in dry pelletized form is then extruded into the tether 12, for example by using a single screw extruder with a string die. Other manufacturing techniques (e.g. molding) known to those of ordinary skill are equally acceptable.

In some embodiments, the tether 12 is formed apart from the personal protective assembly 14, and optionally can incorporate one or more features (e.g., a loop) at one or both of the opposing ends 20a, 20b configured to promote assembly or attach to the personal protective assembly 14. In other embodiments, the opposing ends 20a, 20b do not exhibit any shapes or features differing from a remainder of the tether 12. The tethered personal protective device 10 can be provided to an end user in a final, assembled form (e.g., the tether ends 20a, 20b are pre-attached to the personal protective assembly 14). Alternatively, the tether 12 and the personal protective assembly 14 can be configured for attachment to one another by an end user (e.g., the tether 12 is provided to the end user apart from the personal protective assembly 14). In yet other embodiments, the tether 12 can be integrally formed with the personal protective assembly 14 (e.g., with optional embodiments in which the personal protective assembly 14 includes the earplugs 16a, 16b, the tether 12 can be integrally formed with the earplugs 16a, 16b as a single, homogenous body).

As indicated above, the present disclosure is not limited to a particular type or format of the personal protective assembly 14. With optional embodiment in which the personal protective assembly 14 is or includes the first and second earplugs 16a, 16b, the earplugs 16a, 16b can be identical and can assume various forms. For example, with the exemplary embodiment of FIG. 1, the earplugs 16a, 16b are each roll-down type resilient foam earplugs. Other formats are equally acceptable, such as push-to-fit stem type earplugs, pre-molded polymeric flange type earplugs, or any other desired type of earplug. The first and second ends 20a, 20b are attached to the first and second earplugs 16a, 16b, respectively, for example by ultrasonic welding, adhesive bonding, mechanical bonding, mechanical connection, etc. With embodiments in which the earplugs 16a, 16b are push-to-fit stem type earplugs, the first and second ends 20a, 20b can form, or be formed to generate, a loop for attachment to the corresponding earplug 16a, 16b. Similar attachment techniques can be utilized for the tether 12 with other types of personal protective assemblies 14 (e.g., the first and second ends 20a, 20b can be attached to the opposing temple pieces, respectively; can be attached to various structures provided with a hard hat, etc.).

Regardless of an exact format of the personal protective assembly 14 or how the tether 12 is attached thereto, optional methods of using the tethered personal protective device 10 of the present disclosure include a user receiving the tethered personal protective device 10 with the tether 12 in the initial state (and thus the initial length L1). In some instances, the initial length L1 will be appropriate for, or desired by, the end user, and the end user then simply wears the tethered personal protective device 10 as intended (e.g., inserts the earplugs 16a, 16b into the user's ear canals, respectively, and locates the tether 12 behind the user's neck). In other instances, the user may view the initial length L1 as being too short. Under these circumstances, the user grasps the tether 12 with one hand at or adjacent the first end 20a and with the other hand at or adjacent the second end 20b in the user's normal working environment (e.g., at or about room temperature). The user then applies a tensile or stretching force on to the tether 12, pulling the ends 20a, 20b away from another and causing the tether 12 to stretch to the stretched state, for example by applying a force on the order of 4-5 lbs (18 - 22 N). The stretching process continues until the tether 12 has been stretched to a length desired or selected by the user. In some instances, the user will be satisfied with the so-achieved stretched length L2, and then wears the tethered personal protective device 10 (with the tether 12 self-retaining the stretched length L2). In other instances, the user may find the so-achieved stretched length L2 to be too long. Under these circumstances, the user subjects or exposes the tether 12 to an elevated temperature, for example at least 50 °C (e.g., the user places the tether 12 in a basin or stream of hot water). The tether 12 self-transitions from the stretched state to a recovered state, contracting in length. The user can optionally subject the tether 12 to the elevated temperature for a relatively short period of time (e.g., 30 seconds) so as to control or lessen the level of contraction in length. In some instances, the user will be satisfied with the so-achieved recovered length L3, and then wears the personal protective device as intended (with the tether 12 self-retaining the recovered length L3). In other instances, the user will desire a different tether length, and can subject the tether 12 to one or more stretching and/or recovery operations as described above until the user is ultimately satisfied with the achieved length. Following use of the personal protective device, the user can subject or expose the tether 12 to an elevated temperature as described above, causing the tether 12 to self-transition to a recovered length L3 that approximates, optionally is identical to or even less than, the initial length L1. The tethered personal protective device 10 can then be conveniently stored (with the tether 12 occupying minimal space) for later re-use. In this regard, the tether 12 can later be repeatedly subjected to the stretching and/or recovery operations described above in preparation for the next use.

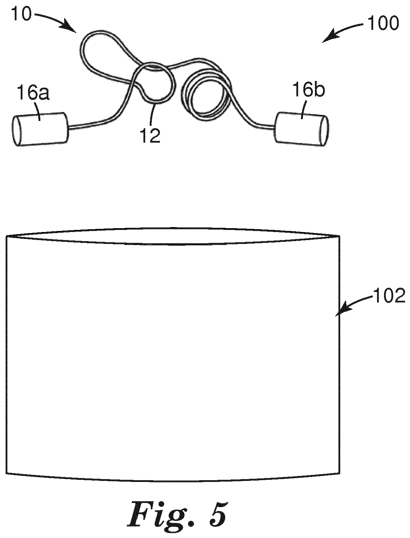

In some embodiments, the tethered personal protective device 10 of the present disclosure can be provided to a user in packaged form. For example, FIG. 5 illustrates one embodiment of a packaged personal protective device article 100 in accordance with principles of the present disclosure, and includes the tethered personal protective device 10 as described above along with a sealed package 102. For ease of explanation, the tethered personal protective device 10 is shown outside of the package 102 in the view of FIG. 5 it being understood that as received by an end user, the tethered personal protective device 10 is sealed within the package 102. With embodiments in which the tether personal protective device 10 is a tethered earplug product, a size of the package 102 can optionally be less than that associated with conventional tethered earplug packaging. For example, some conventional packaged tethered earplug articles incorporate a tether having a length of 24 inches; the corresponding package must be sufficiently large to accommodate this length. A tethered earplug device incorporating the exemplary tether 12 of the present disclosure and having the initial length L1 of, for example, 12 inches (30 cm) and be stretchable to the stretched length L2 of 24 inches (61 cm). Thus, the tethered earplug device incorporating the exemplary tether 12 of the present disclosure can "fit" the same users as the conventional tethered earplug device, yet occupies significantly less space in the initial state; thus, the package 102 can be much smaller, and the manufacturing steps necessary to load the tethered earplug device into the package less complex, as compared to that associated with the conventional tethered earplug device.

**Example and Comparative Examples**

The characteristics, operation, and advantages of the present disclosure will be further described with regard to the following detailed non-limiting examples and comparative examples. These examples and comparative examples are offered to further illustrate the various specific and illustrative embodiments and techniques of the present disclosure. It should be understood, however, that many variations and modifications may be made while remaining within the scope of the present disclosure.

Test Methods

The parameters of stretchability (or ease of stretching), fixity and rate of recovery where determined by carrying out the consecutive testing steps listed below using an Instron 5967 Universal testing instrument.

Tether samples were initially prepared as described below, and cut to an initial length. The initial length and diameter of the tether sample was measured and recorded. The tether sample was placed in the Instron testing instrument by attaching the two instrument clamps to the tether sample. The instrument clamps were spaced by a clamping distance of 50.8 mm, and the location of the clamps were marked on the tether sample. The initial clamping distance of 50.8 mm was designated as the original length $L_O$ for all samples tested. All length measurements were subsequently made with reference to the distance between the two clamp markings on the tether sample.

The testing instrument was operated to subject the tether sample to a stretching or tensile force at room temperature (22.7 °C, 50% RH) at a constant rate (e.g., 300%/minute). The tether sample was stretched to a predetermined length at the constant rate. The predetermined length selected for each tether sample was determined by referencing an expected elongation at break for the material of the particular tether sample. The tether sample was held by the testing instrument at the predetermined length for 3 minutes, and the predetermined length ($L_{MAX}$) was recorded. Following the 3 minutes holding period, the testing instrument was operated to return the clamps to their original position. A maximum load ("Max Load") applied to the tether sample during the stretch and hold operation as reported by the Instron testing instrument was recorded, as was the 100 % modulus as determined by the testing instrument..

Following a 3 minute dwell/recover time period (at room temperature), a length of the stretched tether sample between the two clamp markings was measured and recorded as $L_S$.

The stretched tether sample was then placed in an oven heated to 70 °C (50% RH) for 1 minute. The tether sample was removed from the oven and allowed to dwell/recover at room temperature (22.7 °C, 50% RH) for 5 minutes. Following the 5 minute recovery period, a distance between the clamp markings on the tether sample after heat cycle ($L_{HC}$) was measured and recorded.

Example 1

An exemplary tether in accordance with principles of the present disclosure was formed from a polymer blend of EVA and TPU. The polymer blend of Example 1 included ethylene vinyl acetate (EVA) available from AT Plastics under the trade designation Ateva 2810A (28% VA) and thermoplastic polyurethane (TPU) available from BASF under the trade designation Elastollan 1180A, as well as processing additives of $CaCO_3$ available from Omya under the trade designation Omyacarb 3, Ca stearate available from Spectrum Chemical, and an EVA colorant available from EMC plastics, at the weight percentages listed in Table 1 below. The mixture was processed into a dry pellet form that was then extruded into a tether using a single screw extruder with a string die.

**Table 1**

| Material | Wt% |
| --- | --- |
| EVA | 68 |
| TPU | 20 |
| $CaCO_3$ | 10 |
| Ca stearate | 1 |
| EVA colorant | 1 |

Comparative Examples C1-C3

A first comparative tether sample (C1) was prepared as an extruded length of poly(vinyl chloride). A second comparative tether sample (C2) was prepared as an extruded length of thermoplastic polyurethane (TPU) available from BASF under the trade designation Elastollan 1180A. A third comparative tether sample (C3) was prepared as an extruded length of ethylene vinyl acetate (EVA) available from AT Plastics under the trade designation Ateva 2810A (28% VA).

Results

Each of Example 1 and Comparative Examples C1-C3 were subjected to the testing protocols described above, and results recorded as reported in Table 2.

**Table 2**

| Sample | Original length, $L_O$ (mm) | Original diameter (mm) | Max stretched length $L_{MAX}$ (mm) | Length when stress removed, $L_S$ (mm) | Length after heat cycle, $L_{HS}$ (mm) | Max load (N) | 100% Modulus (MPa) |
| --- | --- | --- | --- | --- | --- | --- | --- |
| Ex. 1 | 50.8 | 1.27 | 355.6 | 222.3 | 104.8 | 34.1 | 3.675 |
| C-1 | 50.8 | 1.27 | 101.6 | 57.15 | 54.61 | 23.6 | 14.5 |
| C-2 | 50.8 | 1.27 | 355.6 | 127 | 93.3 | 68.7 | 3.74 |
| C-3 | 50.8 | 1.27 | 304.8 | 209.55 | 103.12 | 27.8 | 6.53 |

Fixity rate $R_f$, recovery rate $R_R$, and shape memory rate $R_S$ were calculated pursuant to Equations 1-3 above, as were various strain values. The calculated values are reported in Table 3 below.

**Table 3**

| Sample | Fixed strain, $\varepsilon_F$ | Max strain, $\varepsilon_M$ | Heated strain, $\varepsilon_{HC}$ | Fixity rate, $R_f$ | Recover rate, $R_R$ | Shape memory rate, $R_S$ |
|---|---|---|---|---|---|---|
| Ex. 1 | 3.38 | 6.0 | 1.06 | 56% | 53% | 69% |
| C-1 | 0.13 | 1.0 | 0.08 | 13% | 5% | 40% |
| C-2 | 1.50 | 6.0 | 8.4 | 25% | 26% | 44% |
| C-3 | 3.13 | 5.0 | 1.03 | 63% | 51% | 67% |

The testing results show that the exemplary tether of Example 1 performed in accordance with principles of the present disclosure. The exemplary tether of Example 1 was easily stretched, readily self-maintained a stretched length following removal of the stretching force, and self-transitioned to a recovered stated having a contracted length following exposure to heat for a short period of time. The 100% modulus (or yield strength) value is indicative of the ease with which the tether samples could be stretched, with the reported 100% modulus value for Example 1 reflecting that the sample tether could easily be stretched by a user. Moreover, the tether of Example 1 was found to be readily bondable to foam earplugs with an adhesive. It is believed that Comparative Example C-3 (EVA alone) cannot readily bond, if at all, to a foam earplug using an adhesive conventionally employed with tethered foam earplugs.

The tethers, tethered personal protective devices, packaged tethered personal protective device articles, and methods of the present disclosure provide a marked improvement over previous designs. A user is afforded the ability to manually manipulate the tether to virtually any desired length by stretching and/or contracting the tether. Following use, the tether can be contracted to a compact size, promoting ease of storage and re-use. The exemplary tethers of the present disclosure can be shorter than tethers conventionally provided with packaged personal protective devices, thus saving on packaging space and processing steps.

The foregoing detailed description and examples have been given for clarity of understanding only. No unnecessary limitations are to be understood there from. It will be apparent to those skilled in the art that many changes can be made in the embodiments described without departing from the scope of the disclosure. Any feature or characteristic described with respect to any of the above embodiments can be incorporated individually or in combination with any other feature or characteristic, and are presented in the above order and combinations for clarity only. Thus, the scope of the present disclosure should not be limited to the exact details and structures described herein, but rather by the structures described by the language of the claims, and the equivalents of those structures.

Aspects of the invention:

1. A personal protective device tether, comprising:

an elongate tether comprising a polymer blend, the tether defining opposing, first and second ends and a length separating the first and second ends;
wherein the tether is configured to provide an initial state in which the length is an initial length (L1);
and further wherein the tether is configured to self-retain a stretched state when stretched from the initial state at a first temperature (T1), the length in the stretched state being a stretched length (L2) that is greater than L1;
and even further wherein the tether is configured to self-transition from the stretched state to a recovered state when heated to a second temperature (T2) greater than T1, the length in the recovered state being a recovered length (L3) that is less than L2.

2. The tether of aspect 1, wherein T1 is not greater than 50 °C, and further wherein T2 is not less than 60 °C.

3. The tether of aspect 2, wherein T1 is not greater than 30 °C.

4. The tether of aspect 1, wherein L2 is not less than 2x L1, and further wherein L3 is not more than 1.5x L2.

5. The tether of aspect 1, wherein L2 is not less than 3x L1.

6. The tether of aspect 1, wherein L2 is not less than 5x L1, and further wherein L3 is not more than 4x L2.

7. The tether of aspect 6, wherein the tether is configured to self-transition from the stretched state to the recovered state when heated to T2 for a time period of not more than one minute.

8. The tether of aspect 7, wherein T2 is in the range of 60 °C - 80 °C.

9. The tether of aspect 1, wherein the tether exhibits a shape memory rate $R_S$ of at least 60%.

10. The tether of aspect 1, wherein the tether is configured to transition to the stretched state with L2 not less than 5x L1 when subjected to a tensile force of not more than 5 pounds.

11. The tether of aspect 1, wherein the tether exhibits a fixity rate $R_f$ of not less than 50%.

12. The tether of aspect 1, wherein the polymer blend comprises at least two thermoplastic elastomeric polymers.

13. The tether of aspect 1, wherein the polymer blend comprises at least two materials selected from the group consisting of ethylene vinyl acetate (EVA), thermoplastic polyurethane (TPU), thermoplastic olefin (TPO), and styrene-butadiene copolymers.

14. The tether of aspect 1, wherein the polymer blend comprises ethylene vinyl acetate (EVA) and thermoplastic polyurethane (TPU).

15. The tether of aspect 14, wherein the polymer blend comprises between 50 wt % and 95 wt % EVA.

16. The tether of aspect 15, wherein the polymer blend comprises between 5 wt % and 50 wt % TPU.

17. The tether of aspect 16, wherein the polymer blend comprises between 60 wt% and 80 wt% EVA and between 10 wt % and 30 wt % TPU.

18. The tether of aspect 1, further comprising a first hearing protection member attached to the first end.

19. The tether of aspect 18, further comprising a second hearing protection member attached to the second end.

20. The tether of aspect 19, wherein the first and second hearing protection members are earplugs.

21. The tether of aspect 1, wherein the first end is attached to a personal protective device selected from the group consisting of an earplug, eyewear, hard hat, respirator, communication device, and hearing protection device.

22. A personal protective device, comprising:

an elongate tether comprising a polymer blend, the tether defining opposing, first and second ends and a length separating the first and second ends;
wherein the tether is configured to provide an initial state in which the length is an initial length (L1);
and further wherein the tether is configured to self-retain a stretched state when stretched from the initial state at a first temperature (T1), the length in the stretched state being a stretched length (L2) that is greater than L1;
and even further wherein the tether is configured to self-transition from the stretched state to a recovered state when heated to a second temperature (T2) greater than T1, the length in the recovered state being a recovered length (L3) that is less than L2; and
a personal protective assembly attached to the first and second ends.

23. The personal protective device of aspect 22, wherein the personal protective assembly includes a first hearing protection member attached to the first end and a second hearing protection member attached to the second end.

24. The personal protective device of aspect 23, wherein the first and second hearing protection members are each an earplug.

25. A method of using a personal protective device, comprising:

receiving a personal protective device including:

an elongate tether comprising a polymer blend, the tether defining opposing, first and second ends and a length separating the first and second ends;

wherein in the step of receiving includes the tether in an initial state in which the length is an initial length (L1);

applying a stretching force to the tether at a first temperature (T1) to transition the tether to a stretched state;

wherein the length in the stretched state is a stretched length (L2) that is greater than L1, and the tether self-retains the stretched state; and

heating the tether to a second temperature (T2) greater than T1;

wherein the step of heating includes the tether self-transitioning from the stretched state to a recovered state, and further wherein the length in the recovered state is a recovered length (L3) that is less than L2;

wearing the personal protective device with the tether in the stretched state.

26. The method of aspect 25, wherein T1 is less than 50 °C, and further wherein T2 is between 60 °C and 80 °C.

27. The method of aspect 25, wherein the step of heating the tether includes placing the tether in heated water.

28. The method of aspect 25, wherein following the step of heating the tether, the method further comprising:

applying a second stretching force to the tether at a temperature less than T2 to transition the tether to a second stretched state;

wherein the length in the second stretched state is a second stretched length (L4) that is at least 2x L3; and

after the step of applying a second stretching force, heating the tether to a temperature greater than T1;

wherein the step of heating the tether after the step of applying a second stretching force includes the tether self-transitioning from the second stretched state to a second recovered state, and further wherein the length in the second recovered state is a second recovered length (L5) that is less than L4.

29. A packaged personal hearing protection article, comprising:

a personal hearing protection device including:

an elongate tether comprising a polymer blend, the tether defining opposing, first and second ends and a length separating the first and second ends,

wherein the tether is configured to provide an initial state in which the length is an initial length (L1),

and further wherein the tether is configured to self-retain a stretched state when stretched from the initial state at a first temperature (T1), the length in the stretched state being a stretched length (L2) that is greater than L1,

and even further wherein the tether is configured to self-transition from the stretched state to a recovered state when heated to a second temperature (T2) greater than T1, the length in the recovered state being a recovered length (L3) that is less than L2,

a first earplug attached to the first end,

a second earplug attached to the second end; and

a sealed package containing the personal hearing protection device.

**Claims**

1. A personal protective device tether, comprising:

an elongate tether, the tether defining opposing, first and second ends and a length separating the first and second ends;

wherein the tether is configured to provide an initial state in which the length is an initial length (L1);

and further wherein the tether is configured to self-retain a stretched state when stretched from the initial state at a first temperature (T1), the length in the stretched state being a stretched length (L2) that is greater than L1;

and even further wherein the tether is configured to self-transition from the stretched state to a recovered state when heated to a second temperature (T2) greater than T1.

2. The tether of claim 1, wherein the polymer blend comprises between 5 wt % and 50 wt % TPU.

3. The tether of claim 2, wherein the polymer blend comprises between 60 wt% and 80 wt% EVA and between 10 wt % and 30 wt % TPU.

4. The tether of claim 1, further comprising a first hearing protection member attached to the first end.

5. The tether of claim 4, further comprising a second hearing protection member attached to the second end.

6. The tether of claim 5, wherein the first and second hearing protection members are earplugs.

7. The tether of claim 1, wherein the first end is attached to a personal protective device selected from the group consisting of an earplug, eyewear, hard hat, respirator, communication device, and hearing protection device.

8. A personal protective device, comprising:

> an elongate tether, the tether defining opposing, first and second ends and a length separating the first and second ends;
> wherein the tether is configured to provide an initial state in which the length is an initial length (L1);
> and further wherein the tether is configured to self-retain a stretched state when stretched from the initial state at a first temperature (T1), the length in the stretched state being a stretched length (L2) that is greater than L1;
> and even further wherein the tether is configured to self-transition from the stretched state to a recovered state when heated to a second temperature (T2) greater than T1; and
> a personal protective assembly attached to the first and second ends.

9. The personal protective device of claim 8, wherein the personal protective assembly includes a first hearing protection member attached to the first end and a second hearing protection member attached to the second end.

10. The personal protective device of claim 9, wherein the first and second hearing protection members are each an earplug.

11. A method of using a personal protective device, comprising:

> receiving a personal protective device including:

> > an elongate tether, the tether defining opposing, first and second ends and a length separating the first and second ends;
> > wherein in the step of receiving includes the tether in an initial state in which the length is an initial length (L1);

> applying a stretching force to the tether at a first temperature (T1) to transition the tether to a stretched state;
> wherein the length in the stretched state is a stretched length (L2) that is greater than L1, and the tether self-retains the stretched state; and
> heating the tether to a second temperature (T2) greater than T1;
> wherein the step of heating includes the tether self-transitioning from the stretched state to a recovered state;
> wearing the personal protective device with the tether in the stretched state.

12. The method of claim 11, wherein T1 is less than 50 °C, and further wherein T2 is between 60 °C and 80 °C.

13. The method of claim 11, wherein the step of heating the tether includes placing the tether in heated water.

14. The method of claim 11, wherein following the step of heating the tether, the method further comprising:

> applying a second stretching force to the tether at a temperature less than T2 to transition the tether to a second stretched state;
> wherein the length in the second stretched state is a second stretched length (L4) that is at least 2x L3; and
> after the step of applying a second stretching force, heating the tether to a temperature greater than T1;
> wherein the step of heating the tether after the step of applying a second stretching force includes the tether self-transitioning from the second stretched state to a second recovered state, and further wherein the length in the second recovered state is a second recovered length (L5) that is less than L4.

**15.** A packaged personal hearing protection article, comprising:

a personal hearing protection device including:

an elongate tether, the tether defining opposing, first and second ends and a length separating the first and second ends,
wherein the tether is configured to provide an initial state in which the length is an initial length (L1),
and further wherein the tether is configured to self-retain a stretched state when stretched from the initial state at a first temperature (T1), the length in the stretched state being a stretched length (L2) that is greater than L1,
and even further wherein the tether is configured to self-transition from the stretched state to a recovered state when heated to a second temperature (T2) greater than T1,
a first earplug attached to the first end,
a second earplug attached to the second end; and

a sealed package containing the personal hearing protection device.

10

16b

16a

14

12

20b

20a

14

*Fig. 1*

20b                          12                    20a

L1

*Fig. 2*

F    20b                      12                  20a    F

L2

*Fig. 3*

20b                          12                    20a

L3

*Fig. 4*

*Fig. 5*

Europäisches
Patentamt

European
Patent Office

Office européen
des brevets

# EUROPEAN SEARCH REPORT

Application Number

EP 18 16 8983

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | US 2005/230181 A1 (WOO EDWIN [US]) 20 October 2005 (2005-10-20) * paragraph [0011] - paragraph [0019]; figure 1 * ----- | 1-15 | INV. A61F11/12 |
| A,D | US 5 668 354 A (FALCO ROBERT N [US]) 16 September 1997 (1997-09-16) * column 2, line 29 - line 60; figure 1 * ----- | 1-15 | |

TECHNICAL FIELDS
SEARCHED      (IPC)

A61F
H04R

The present search report has been drawn up for all claims

| Place of search | Date of completion of the search | Examiner |
|---|---|---|
| Munich | 31 May 2018 | Skorovs, Peteris |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

# EP 3 375 418 A1

ANNEX TO THE EUROPEAN SEARCH REPORT
ON EUROPEAN PATENT APPLICATION NO.

EP 18 16 8983

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2018

| Patent document cited in search report | Publication date | Patent family member(s) | Publication date |
|---|---|---|---|
| US 2005230181 A1 | 20-10-2005 | US 2005230181 A1<br>WO 2005107321 A2 | 20-10-2005<br>10-11-2005 |
| US 5668354 A | 16-09-1997 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5668354 A, Falco
- US 20050230181 A, Woo